(19) Europäisches Patentamt
European Patent Office
Office européen des brevets

(11) **EP 4 298 989 A1**

(12) **EUROPEAN PATENT APPLICATION**

(43) Date of publication:
**03.01.2024 Bulletin 2024/01**

(21) Application number: **22182245.5**

(22) Date of filing: **30.06.2022**

(51) International Patent Classification (IPC):
**A61B 5/00** *(2006.01)*    **A61B 7/00** *(2006.01)*

(52) Cooperative Patent Classification (CPC):
**A61B 5/4815; A61B 5/4818; A61B 7/003**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **LONG, Xi**
**Eindhoven (NL)**

• **XIE, Jiali**
**Eindhoven (NL)**
• **DOS SANTOS DA FONSECA, Pedro Miguel Ferreira**
**5656AG Eindhoven (NL)**
• **VAN DIJK, Hans**
**5656AG Eindhoven (NL)**

(74) Representative: **Philips Intellectual Property & Standards**
**High Tech Campus 52**
**5656 AG Eindhoven (NL)**

(54) **SYSTEM AND METHOD FOR DETERMINING A MEASURE OF A SEVERITY OF SLEEP DISORDERED BREATHING**

(57)    A system and method for determining a measure of a severity of sleep disordered breathing for a subject. A time-varying signal responsive to the subject's breathing during a sleep session is obtained from a breathing detector, and processed to determine characteristics representative of a variation in snore-to-snore intervals. These characteristics are processed to determine the measure of the severity of sleep disordered breathing.

FIG. 1

**Description**

FIELD OF THE INVENTION

The invention relates to the field of obstructive sleep apnea, and in particular to measuring a severity of sleep disordered breathing.

BACKGROUND OF THE INVENTION

Obstructive sleep apnea (OSA) is an increasingly common sleep disorder, affecting between 9% and 17% of the general adult population.

Polysomnography (PSG) is the gold standard for diagnosing OSA. However, this method of detecting OSA requires expensive, specialist equipment (generally at sleep center facilities that require overnight hospitalization), and the instruments used in PSG can disrupt sleep.

Other methods for detecting/determining a severity of OSA (e.g. based on electrocardiography, photoplethysmography or oxygen saturation measurements) also require specialist sensors, and often involve the use of sensors that require attachment to a subject's body, which can cause discomfort.

These difficulties also apply to the determination of a severity of other types of sleep disordered breathing, such as central sleep apnea and upper airway resistance syndrome.

There is therefore a need for a less obtrusive and less costly method for determining the severity of OSA and other sleep disordered breathing (SDB) conditions.

SUMMARY OF THE INVENTION

The invention is defined by the claims.

According to examples in accordance with an aspect of the invention, there is provided a processing system for determining a measure of a severity of sleep disordered breathing, the processing system being configured to: obtain, from a first breathing detector, a first time-varying signal responsive to a subject's breathing during a sleep session of the subject; process the first time-varying signal to determine one or more snore rate variability characteristics; and process at least the one or more snore rate variability characteristics to determine a measure of a severity of sleep disordered breathing.

This method enables the determination of sleep disordered breathing (SDB) severity (OSA) from data that may be obtained easily and unobtrusively. A time-varying signal responsive to breathing may be obtained using a variety of sensors, including microphones on smartphones, tablet computers etc., allowing the severity of sleep disordered breathing to be determined without the need for specialist equipment.

Snore rate variability characteristics are characteristics of a variation in snore-to-snore intervals. The inventors have recognized that snoring is a common symptom of OSA and other types of sleep disordered breathing, and that the variation in snore-to-snore intervals is characteristic of the severity of sleep disordered breathing.

The one or more snore rate variability characteristics may include time domain features, frequency domain features, non-linear features, visibility graph features, spectral features, entropy features, DFA features, Teager energy features, (self-)similarity features, correlation dimension measure, recurrence plot features, spatial filling index, and central tendency measure. Preferably, the one or more snore rate variability includes at least one of a time domain feature and/or a non-linear feature.

The processing system may use a machine learning algorithm to determine the measure of SDB severity. Alternatively, the processing system may use heuristic methods based on thresholds for the one or more snore rate variability characteristics.

In some examples, the first breathing detector comprises at least one of: a microphone, a nasal pressure sensor, a polvinylidene fluoride sensor, a stretchable strain sensor, an accelerometer, a gyroscope and/or a ballistocardiographic sensor.

In some examples, the processing system is configured to determine the one or more snore rate variability characteristics by: processing the first time-varying signal to detect a first plurality of snores in the first time-varying signal; determining a time interval between consecutive snores in the first plurality of snores; and processing the time intervals between consecutive snores to determine the one or more snore rate variability characteristics.

In some examples, the time intervals used to determine the snore rate variability characteristics comprise only time intervals between consecutive snores that do not exceed a predetermined time interval threshold.

This excludes intervals between snoring sessions (i.e. periods of continuous or near-continuous snoring) from the snore rate variability, as the time between stopping snoring and starting again is not characteristic of SDB severity and would skew the output of the determination of the measure severity if included in the snore rate variability.

For example, consecutive snores that are separated by an interval of more than 60 seconds may be considered to belong to different snoring sessions.

In some examples, the first breathing detector comprises a microphone; the processing system is further configured to: obtain, from a second breathing detector, a second time-varying signal responsive to a subject's breathing during a sleep session of the subject, wherein the second breathing detector comprises a microphone located at a different position to the first breathing detector; process the second time-varying signal to detect a second plurality of snores in the second time-varying signal; and process the first and second plurality of snores to determine a location of each snore; and the consecutive snores between which the processing system is configured to determine a time interval are consecutive snores corresponding to a same desired location.

The location of a snore may be determined based on the time difference between when the snore is detected in the first time-varying signal and when the same snore is detected in the second time-varying signal.

Excluding snores that do not come from a desired location reduces the likelihood of the snore rate variability including intervals between false detected snores (i.e. snores that do not belong to the subject). False snores may be detected if the subject has a bed partner who also snores.

In some examples, the processing system is further configured to: obtain, from a third breathing detector, a third time-varying signal responsive to a subject's breathing during a sleep session of the subject, wherein the first breathing detector and third breathing detector are different types of breathing detector; process the third time-varying signal to detect a third plurality of snores in the third time-varying signal; and for each snore in the first plurality of snores, determine whether the snore corresponds to a snore in the third plurality of snores; and the consecutive snores between which the processing system is configured to determine a time interval are consecutive snores that each correspond to a snore in the third plurality of snores.

This provides another mechanism by which false snores may be excluded: if a snore is detected by only one of two detectors, it is likely to be a false snore.

A snore in the first plurality of snores may be considered to correspond to a snore in the third plurality of snores if a snore in the third plurality of snores occurs within a predetermined period of the time at which the snore in the first plurality of snores occurred.

In some examples, the processing system is further configured to: process the first time-varying signal to determine one or more snore energy characteristics, wherein the one or more snore energy characteristics comprise one or more characteristics of at least one measure of snore energy; and determine the measure of the severity of sleep disordered breathing by processing at least the one or more snore rate variability characteristics and the one or more snore energy characteristics.

The at least one measure of snore energy may be a measure of a magnitude of snore energy and/or a gradient of snore energy with respect to time. A measure of a magnitude of snore energy may, for example, be a root mean square or amplitude of the first time-varying signal.

The one or more characteristics for each measure may include a percentage of the measure that is above a predetermined value, a percentile of the measure and/or statistical distribution parameters (e.g. mean, variance, standard deviation, skewness, kurtosis, minimum, maximum, median and/or interquartile range).

The inventors have found that the energy of snore events has increases or decreases during or near some apnea and hypopnea events, while this is not the case for regular snoring. Parameters of trends in snore energy may therefore be used to improve an accuracy of the SDB severity determination.

Parameters of whole night snore energy may also be characteristic of SDB severity and may be additionally used in the determination of the measure of SDB severity.

In some examples, the processing system is further configured to: obtain one or more physical and/or demographic characteristics for the subject; and determine the measure of the severity of sleep disordered breathing by processing at least the one or more snore rate variability characteristics and the one or more physical and/or demographic characteristics.

The one or more demographic characteristics may comprise, for example, one or more of age, body mass index (BMI) and/or sex.

The one or more physical characteristics may comprise, for example, a measure of daytime physical activity, a neck circumference, and/or a diagnosis of a medical condition (e.g. hypertension, comorbid cardiac conditions, etc.)

These characteristics may be additionally used to further improve an accuracy of the measure of SDB severity.

In some examples, the measure of SDB severity may be further based on one or more subjective indicators obtained via user input. The one or more subjective indicators may comprise at least one of: a measure of daytime sleepiness, a reported occurrence of snoring and/or a breathing pause during a previous sleep session (e.g. reported by the subject's bed partner), a reported occurrence of a difficulty initiating or maintaining sleep during a previous sleep session, a measure of stress, and/or a reported occurrence of a difficulty in concentrating.

In some examples, the processing system is further configured to: obtain, from a sleep-monitoring device, sleep data representative of a sleep quality of the sleep session of the subject; process the sleep data to determine one or more sleep quality characteristics of the sleep session; determine the measure of the severity of sleep disordered breathing by processing at least the one or more snore rate variability characteristics and the one or more sleep quality characteristics.

Sleep-monitoring devices (or sleep trackers) are commonly used by subjects with poor sleep quality (including subjects having OSA) to provide data representative of sleep quality (e.g. movement data, heart rate data and/or respiration data). The inventors have recognized that this data may be used to further improve an accuracy of the SDB severity determination.

The one or more sleep quality characteristics may comprise one or more of: a total time spent sleeping, a time spent in a particular sleep stage, a percentage of the sleep session spent in a particular sleep stage, a number of sleep stage transitions during the sleep session, a rate of sleep stage transitions during the sleep session, a number of arousals during the sleep session, a rate of arousals during the sleep session, a number of awakenings during the sleep session, a rate of awakenings during the sleep session, and/or a characteristic (e.g. average entropy) derived from a hypnodensity (the probability of different sleep stages per epoch or sleep session).

In some examples, the processing system is further configured to: process the first time-varying signal to determine one or more further snoring characteristics, wherein the one or more further snoring characteristics comprises at least one of: an inter event silence, a Mel cepstability, a Mel-frequency cepstral coefficient, an apneic phase ratio, a spectral power density and a running variance for a time interval between consecutive snores; and determine the measure of the severity of sleep disordered breathing by processing at least the one or more snore rate variability characteristics and the one or more further snoring characteristics.

These characteristics may further improve an accuracy of the SDB severity determination.

In some examples, the processing system is configured to determine the measure of the severity of sleep disordered breathing using a machine-learning algorithm, wherein: the machine-learning algorithm has been trained using a training algorithm configured to receive an array of training inputs and known outputs, each training input corresponding to a respective known output; each training input comprises at least one or more snore rate variability characteristics for a sleep session for a subject; and each known output comprises a measure of a severity of sleep disordered breathing for the subject.

In some examples, the training inputs may additionally comprise one or more of snore energy characteristics, demographic characteristics, sleep quality characteristics and further snoring characteristics.

In some examples, the determined measure of the severity of sleep disordered breathing is a severity level selected from a plurality of predefined severity levels, an estimated apnea hypopnea index, an estimated obstructive apnea index, an estimated hypopnea index, an estimated central apnea index, an estimated oxygen desaturation index, or an estimated respiratory disturbance index.

For example, the predetermined severity levels may comprise "mild and normal", "moderate" and "severe".

Various measures of the severity of sleep disordered breathing are used in clinical practice. For example, the apnea hypopnea index (AHI) may be used where the measure of the severity of sleep disordered breathing is a measure of obstructive sleep apnea (OSA) severity. AHI is a number of apnea or hypopnea events per hour during a sleep session, and is a common measure of an OSA severity. AHI is generally determined using polysomnography. A higher AHI indicates a more severe level of OSA.

AHI may be decomposed into its parts: the obstructive apnea index, the hypopnea index, and the central apnea index. One or more of these indices may be used individually as a measure of OSA severity.

The oxygen desaturation index (ODI) is often used as a surrogate measure for AHI when only SpO2 measurements are available, while the respiratory disturbance index (RDI) is considered a more complete measure of OSA severity than AHI, as it additionally includes a measurement of respiratory-effort related arousals (RERAs).

There is also proposed a system for determining a measure of a severity of sleep disordered breathing, the system comprising a breathing detector capable of obtaining a time-varying signal responsive to a subject's breathing during a sleep session of the subject, and the processing system described above.

According to another aspect of the invention, there is provided a computer-implemented method for determining a measure of a severity of sleep disordered breathing, the computer-implemented method comprising: obtaining, from a first breathing detector, a first time-varying signal responsive to a subject's breathing during a sleep session of the subject; processing the first time-varying signal to determine one or more snore rate variability characteristics; and processing at least the one or more snore rate variability characteristics to determine a measure of a severity of sleep disordered breathing.

There is also proposed a computer program product comprising computer program code means which, when executed on a computer device having a processing system, cause the processing system to perform all of the steps of the method described above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates a system for determining a measure of a severity of sleep disordered breathing, according to an embodiment of the invention;
Figure 2 illustrates an example time-varying signal 200 responsive to the breathing of a subject during a sleep session;
Figure 3 illustrates a graph of the times of detected snore sounds for a subject during a part of a sleep session, alongside a graph of the time intervals between consecutive snores in each snore group;
Figure 4 illustrates a scatter plot of a percentage of successive intervals between consecutive snores that differ by more than 70 ms against apnea hypopnea index for a group of subjects;
Figure 5 illustrates a scatter plot of a percentage of the standard deviation of successive snore-to-snore intervals larger than a standard deviation threshold against apnea hypopnea index for the same group of subjects;
Figure 6 illustrates a scatter plot of an SRV triangular index against apnea hypopnea index for the same group of subjects;
Figure 7 illustrates a scatter plot of a low frequency relative power against apnea hypopnea index for the same group of subjects;
Figure 8 illustrates an example time-varying signal for a subject during a series of hypopnea events;
Figure 9 illustrates a confusion matrix of a 5-fold cross-validation of a first trained support-vector machine classifier;
Figure 10 illustrates a confusion matrix of a 5-fold cross-validation of a first trained support-vector machine classifier;
Figure 11 illustrates a scatter plot of estimated AHI from a linear regression model against reference AHI from polysomnography;
Figure 12 illustrates a Bland-Altman plot of reference AHI and estimated AHI from the linear regression model; and
Figure 13 illustrates a computer-implemented method 1300 for determining a measure of a severity of sleep disordered breathing, according to an embodiment of the invention.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
There is proposed a system and method for determining a measure of a severity of sleep disordered breathing for a subject. A time-varying signal responsive to the subject's breathing during a sleep session is obtained from a breathing detector, and processed to determine characteristics representative of a variation in snore-to-snore intervals. These characteristics are processed to determine the measure of the severity of sleep disordered breathing.
Embodiments are at least partly based on the realization that the variation in intervals between consecutive snores during a period of snoring is characteristic of a severity of sleep disordered breathing, and that characteristics representative of this variation may be determined easily and unobtrusively by obtaining a signal responsive to a subject's breathing.
Illustrative embodiments may, for example, be employed in sleep monitoring applications (e.g. for a smartphone), wearable devices for sleep monitoring/sleep disordered breathing monitoring and any other systems and devices used to monitor sleep disordered breathing and/or estimate a severity of sleep disordered breathing.

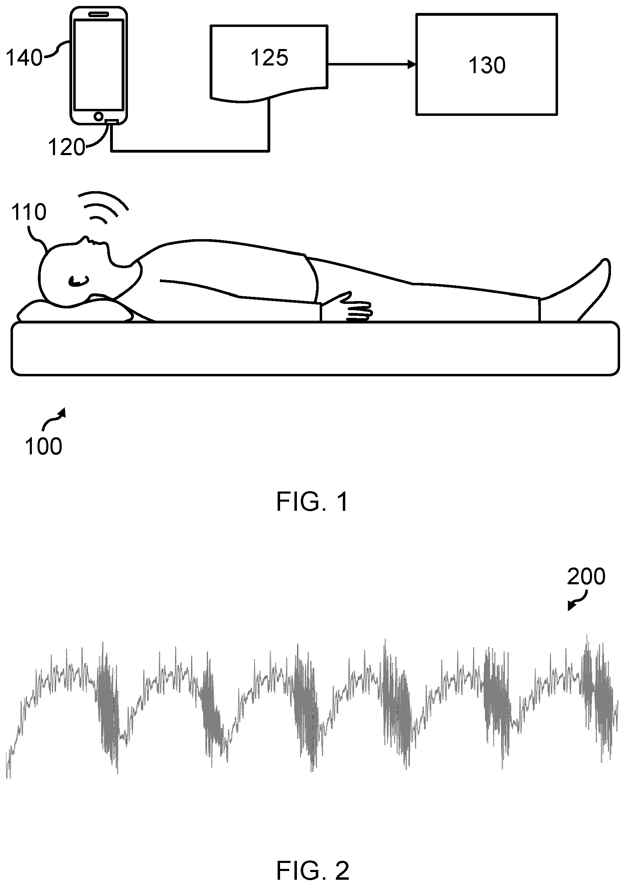

Figure 1 illustrates a system 100 for determining a measure of a severity of sleep disordered breathing (SDB) for a subject 110, according to an embodiment of the invention. The system 100 comprises a first breathing detector 120 and a processing system 130. The processing system is, itself, an embodiment of the invention.
In Figure 1, the first breathing detector 120 is a microphone of a smartphone 140 located close to the subject 110 during the sleeping session (e.g. on a night table by the subject's bed); however, the first breathing detector may be any detector capable of obtaining a time-varying signal responsive to a subject's breathing during a sleep session of the subject. The detector may detect breathing sounds (i.e. as an audio signal), or vibrations or other movements associated with breathing.
For example, the first breathing detector 120 may comprise at least one of: a microphone (e.g. a contact microphone placed on/against the subject 110, such as on the subject's neck, or a non-contact microphone in the vicinity of the subject, i.e. close enough to detect breathing/snoring sounds, e.g. within 1 m of the subject), a resonator, a pressure sensor (e.g. a nasal pressure sensor or a stretchable strain sensor), an accelerometer, a gyroscope, and/or a ballistocardiographic sensor (e.g. a polyvinylidene sensor). The use of such detectors to obtain a signal responsive to a subject's breathing is well known. Further examples of suitable detectors for obtaining a time-varying signal responsive to a subject's breathing will be apparent to the skilled person.

The first breathing detector 120 obtains a first time-varying signal 125 responsive to the breathing of the subject 110 during a sleep session of the subject. Figure 2 illustrates an example time-varying signal 200 responsive to the breathing of a subject during a sleep session. The example time-varying signal 200 was obtained by an accelerometer mounted on a belt worn around the subject's thorax. The low frequency amplitude variations in the signal 200 correspond to regular breathing movements, the peak complexes overlaid on the respiratory variations correspond to the seismocardiographic effect of the beating heart, and the high frequency periods at the end of each breathing cycle correspond to snores.

Returning to Figure 1, the processing system 130 obtains the first time-varying signal 125 from the first breathing detector 120, and processes the first time-varying signal to determine one or more snore rate variability (SRV) characteristics.

The processing system 130 may determine the one or more snore rate variability characteristics by processing the first time-varying signal 125 to detect a first plurality of snores in the first time-varying signal, determining a time interval between consecutive snores in the first plurality of snores, and processing the time intervals between consecutive snores to determine the one or more snore rate variability characteristics.

Methods for detecting a plurality of snores in a time-varying signal responsive to breathing are well known, and suitable methods for processing the first time-varying signal will be apparent to the skilled person. See, for example: Jiali Xie et al. (2021), "Audio-based snore detection using deep neural networks", Computer Methods and Programs in Biomedicine, 200:105917; Eliran Dafna et al. (2013), "Automatic detection of whole night snoring events using non-contact microphone", PloS One, 8(12):e84139; Erna Arnardottir et al. (2016), "How to measure snoring? A comparison of the microphone, cannula and piezoelectric sensor", Journal of Sleep Research, 25(2):158-168; D Sanchez Morillo et al. (2007), "Monitoring and analysis of cardio respiratory and snoring signals by using an accelerometer", Annual International Conference of the IEEE Engineering in Medicine and Biology Society, 2007:3942-5; Hyo-Ki Lee et al. (2013), "Automatic snoring detection from nasal pressure data", Annual International Conference of the IEEE Engineering in Medicine and Biology Society, 2013:6870-2; and Su Hwan Hwang et al. (2015), "Polyvinylidene fluoride sensor-based method for unconstrained snoring detection", Physiological Measurement, 36(7):1399-1414.

In some examples, the time intervals used to determine the snore rate variability characteristics comprise only time intervals between consecutive snores that do not exceed a predetermined time interval threshold. In other words, the snores may be grouped into snore groups according to the length of time interval between consecutive snores, with a snore occurring a long time (i.e. longer than the time interval threshold) after an immediately preceding snore belonging to a separate snore group to the immediately preceding snore. If a time interval between consecutive snores does not exceed the predetermined time interval threshold, the snores may be assigned to a same snore group. The predetermined time interval threshold may, for example, have a value between 30 seconds and 120 seconds (e.g. 60 seconds).

The time intervals between consecutive snores belonging to a same snore group may then be processed to determine the snore rate variability characteristics, while the time intervals between snore group are not used in the determination of the snore rate variability characteristics. In this way, intervals between separate periods of snoring, which are not characteristic of a severity of sleep disordered breathing, are not used when determining the measure of SDB severity.

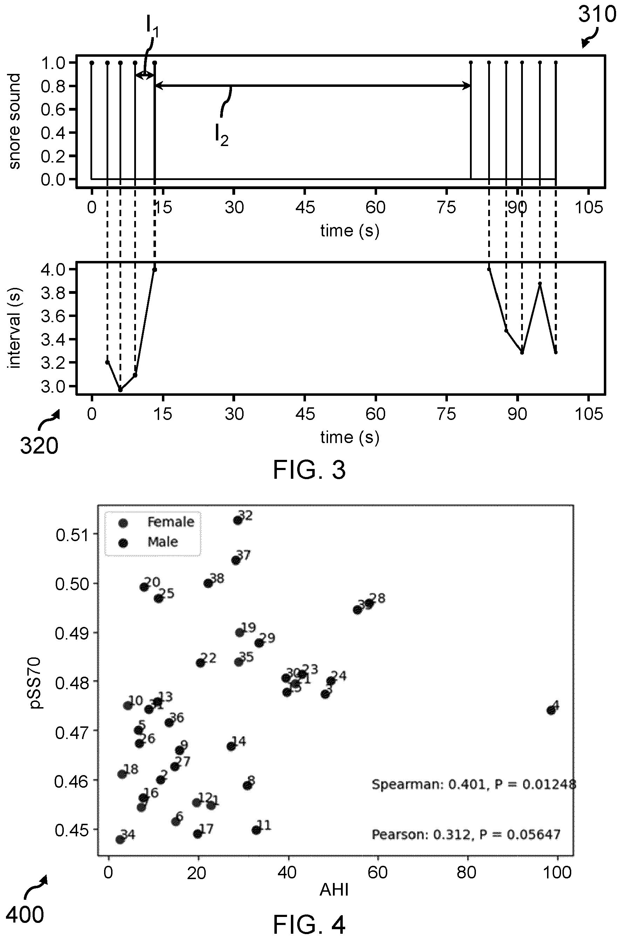

This concept is illustrated by Figure 3, which shows a graph 310 of the times of detected snore sounds for a subject during a part of a sleep session, alongside a graph 320 of the time intervals between consecutive snores in each snore group. In the example shown in Figure 3, consecutive snores having a time interval between them of 60 second or less (such as interval $I_1$) are grouped into the same snore group, while consecutive snores having a time interval between them greater than 60 seconds (such as interval $I_2$) belong to different snore group.

Returning to Figure 1, in some examples, the processing system 130 may additionally apply rules relating to a number of snores in each snore group when grouping the snores (e.g. a minimum and/or a maximum number of snores). For instance, the processing system may group the snores such that each snore group contains at least 5 snores and at most 20 snores.

The determined one or more snore rate variability characteristics are characteristics related to a variation in snore-to-snore intervals (i.e. intervals between consecutive snores). In other words, the snore rate variability features may be variability features of a series of time intervals between consecutive snores (e.g. the graph 320 of Figure 3). The series may exclude intervals between snore groups (i.e. the series of snore-to-snore intervals for an entire sleep session may be obtained by concatenating the series of snore-to-snore intervals for all snore groups in the sleep session).

Tables I to IV list some example snore rate variability characteristics that may be used to determine a measure of SDB severity: Table I provides example time domain features; Table II provides example frequency domain features;

Table III provides example non-linear features; and Table IV provides example visibility graph features.

TABLE I

| Parameter | Unit | Definition |
|---|---|---|
| SDSS | ms | Standard deviation of SS (snore-to-snore) intervals (i.e. intervals between consecutive snores) |
| SDASS | ms | Standard deviation of the average SS intervals for each snore group of an entire sleep session |
| SDSS index | ms | Mean of the standard deviations of all SS intervals for each snore group of an entire sleep session |
| pSS70 | % | Percentage of successive SS intervals that differ by more than 70 ms |
| RMSSD | ms | Root mean square of successive SS interval differences |
| SDSD | ms | Standard deviation of successive SS interval differences |
| SDSS ratio | % | Percentage of SDSS larger than a certain threshold which is determined by the maximum and minimum of SDSS (e.g. determined based on training set data) |
| SRV triangular index | - | Integral of the density of the SS interval histogram divided by its height |
| TISS | - | Triangular interpolation of the SS interval histogram: the baseline width of the distribution measured as a base of a triangle, approximating the SS interval distribution |
| SDSSG | ms | Standard deviation of SS intervals of each snore group |
| ASSG | ms | Average SS intervals of each snore group |
| RMSSDG | ms | Root mean square of successive SS interval differences of each snore group |
| SDSDG | ms | Standard deviation of successive SS interval differences of each snore group |
| SS_Max_SS_Min | ms | The difference between the maximum SS interval and the minimum SS interval of each snore group |

TABLE II

| Parameter | Unit | Definition |
|---|---|---|
| VLF relative power | % | Relative power of the very-low-frequency band (0.0006-0.008 Hz) |
| LF relative power | % | Relative power of the low-frequency band (0.008-0.03 Hz) |
| HF relative power | % | Relative power of the high-frequency band (0.03-0.08 Hz) |
| LF/HF | % | Ratio of low-frequency to high-frequency power |

TABLE III

| Parameter | Unit | Definition |
|---|---|---|
| S | ms | Area of the ellipse which represents total snore rate variability (SRV), where the total snore rate variability is the series of snoreto-snore intervals for an entire sleep session (obtained by concatenating the series of snore-to-snore intervals for all snore groups in the sleep session) |
| SD1 | ms | Poincare plot standard deviation perpendicular to the line of identity |
| SD2 | ms | Poincare plot standard deviation along the line of identity |
| SD 1/SD2 | % | Ratio of SD1 to SD2 |
| DFA $\alpha 1$ | - | Detrended fluctuation analysis, which describes short-term fluctuations of SRV |
| DFA $\alpha 2$ | - | Detrended fluctuation analysis, which describes long-term fluctuations of SRV |

(continued)

| Parameter | Unit | Definition |
|---|---|---|
| SampEn | - | Sample entropy, which measures the regularity and complexity of a time series |
| MSE | - | Multiscale entropy, which measures the complexity of fluctuations over a range of time series |

TABLE IV

| Parameter | Unit | Definition |
|---|---|---|
| $D_m$ | - | Mean of degree (the number of edges attached to a node) |
| $D_{sd}$ | - | Standard deviation of degree |
| CCm | - | Mean of clustering coefficient which measures the density of local clusters in a network |
| $CC_{sd}$ | - | Standard deviation of clustering coefficient |
| ACm | - | Means of assortativity coefficient which represents the skewness of node connections |
| $AC_{sd}$ | - | Standard deviation of assortativity coefficient |

However, it should be understood that the one or more snore rate variability characteristics are not limited to the examples given in Tables I to IV, and may including any features that describe a variation or variability in snoring, such as any variability features in the time domain, frequency domain features (i.e. spectral features), non-linear features and/or visibility graph (VG) features. Non-linear features may include entropy features, detrended fluctuation analysis (DFA) features, Teager energy features, (self-)similarity features, correlation dimension measure, recurrence plot features (e.g. Poincare plot features), a spatial filling index, and/or a central tendency measure. In some examples, the one or more snore rate variability characteristics may include at least one time domain feature and/or at least one non-linear feature. These are commonly-used features for analyzing a time series, and methods for determining these features from a time series of intervals between consecutive snores will be apparent to the skilled person.

In some examples, the one or more snore rate variability characteristics may include a percentage of successive snore-to-snore intervals (i.e. intervals between consecutive snores) that differ by more than 70 ms (pSS70). Figure 4 illustrates a scatter plot 400 of pSS70 (provided in decimal format in Figure 4) against AHI (apnea hypopnea index) for a group of subjects (including both male and female subjects). As Figure 4 shows, the percentage of successive intervals between consecutive snores that differ by more than 70 ms for a subject during a sleep session correlates with the subject's AHI during the sleep session, having a Spearman's rank correlation coefficient of 0.401 (P value = 0.01248) and a Pearson correlation coefficient of 0.312 (P value = 0.05647).

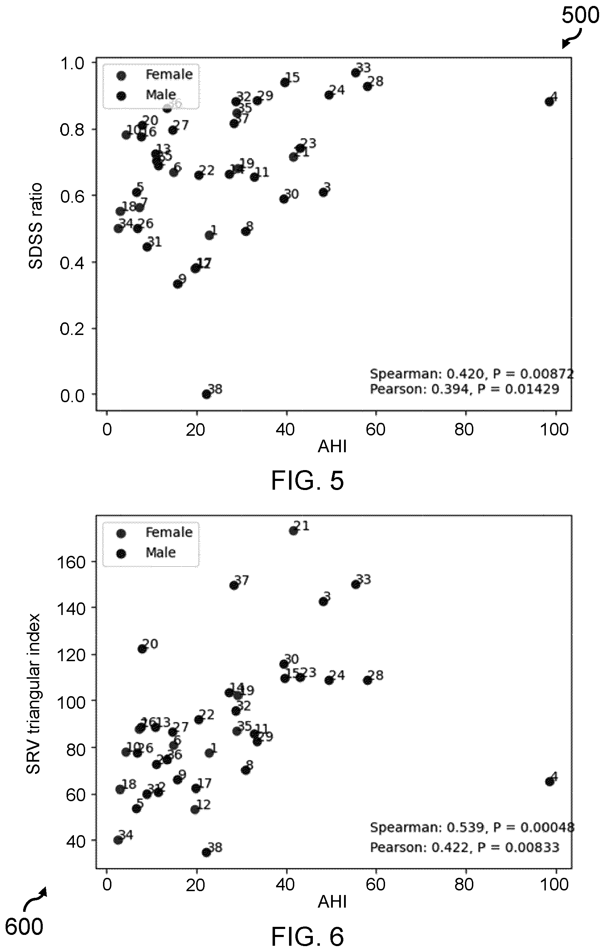

In some examples, the one or more snore rate variability characteristics may include an SDSS ratio (defined as a percentage of the standard deviation of successive snore-to-snore intervals larger than a standard deviation threshold, where the standard deviation threshold is determined by the maximum and minimum of the standard deviation of successive snore-to-snore intervals). Figure 5 illustrates a scatter plot 500 of SDSS ratio (provided in decimal format in Figure 5) against AHI for the same group of subjects used to produce Figure 4. The standard deviation threshold used to determine the SDSS ratio in Figure 5 was determined by maximizing correlation between the standard deviation and AHI. As Figure 5 shows, the SDSS ratio for a subject during a sleep session is also correlated with the subject's AHI during the sleep session, having a Spearman's rank correlation coefficient of 0.420 (P value = 0.00872) and a Pearson correlation coefficient of 0.394 (P value = 0.01429).

In some examples, the one or more snore rate variability characteristics may include an SRV triangular index (defined as an integral of a density of a snore-to-snore interval histogram divided by its height). Figure 6 illustrates a scatter plot 600 of SRV triangular index against AHI for the same group of subjects used to produce Figures 4 and 5. As Figure 6 shows, the SRV triangular index for a subject during a sleep session is also correlated with the subject's AHI during the sleep session, having a Spearman's rank correlation coefficient of 0.539 (P value = 0.00048) and a Pearson correlation coefficient of 0.422 (P value = 0.00833).

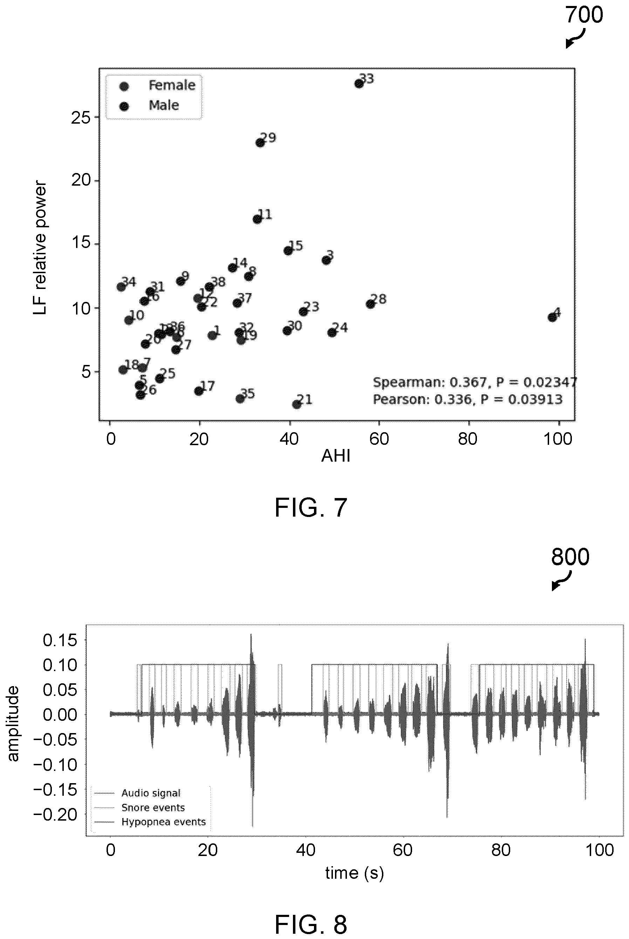

In some examples, the one or more snore rate variability characteristics may include a relative power of the low-frequency band (0.008-0.03 Hz) (LF relative power). Figure 7 illustrates a scatter plot 500 of LF relative power against AHI for the same group of subjects used to produce Figures 4 to 6. As Figure 7 shows, the LF relative power for a subject during a sleep session is also correlated with the subject's AHI during the sleep session, having a Spearman's rank correlation coefficient of 0.367 (P value = 0.02347) and a Pearson correlation coefficient of 0.336 (P value = 0.03913).

Returning to Figure 1, having determined the one or more snore rate variability characteristics, the processing system 130 then processes at least the one or more snore rate variability characteristics to determine a measure of SDB severity.

The measure of SDB severity may, for example, be a severity level for a sleep disordered breathing condition (e.g. an OSA severity) selected from a plurality of predefined severity levels (e.g. "mild and normal", "moderate" and "severe", or "normal", "mild", "moderate" and "severe"). In clinical practice, an OSA severity is typically considered normal in subjects having an AHI < 5, mild in subjects having an AHI greater than or equal to 5 and less than 15, moderate in subjects having an AHI greater than or equal to 15 and less than 30, and severe in subjects having an AHI greater than or equal to 30. However, other thresholds may also be used to determine SDB severity: for instance, when analyzing the residual AHI measured during CPAP therapy, an AHI cutoff of 10 is often used (with an AHI consistently above 10 indicating that therapy may be insufficient to treat the patient).

Where the sleep disordered breathing condition is obstructive sleep apnea (OSA), the measure of SDB severity may be an estimated apnea hypopnea index (AHI). The measure of SDB severity may alternatively be any other measures of SDB severity used in clinical practice, such as an estimated obstructive apnea index, an estimated hypopnea index, an estimated central apnea index, an estimated oxygen desaturation index, or an estimated respiratory disturbance index.

In some examples, the processing system 130 may determine the measure of SDB severity using a heuristic approach. For example, the measure of SDB severity may be determined using a set of predetermined rules, such as rules relating to thresholds for each of the one or more snore rate variability characteristics.

In some examples, the processing system 130 may determine the measure of SDB severity by processing at least the one or more snore rate variability characteristics using a machine-learning algorithm.

A machine-learning algorithm is any self-training algorithm that processes input data in order to produce or predict output data. Here, the input data comprises at least the one or more snore rate variability characteristics and the output data comprises a measure of SDB severity.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example snore rate variability characteristics. The training output data entries correspond to measures of SDB severity.

In some examples, the processing system 130 may use additional characteristics of the subject (i.e. in addition to the snore rate variability characteristics) to determine the measure of SDB severity. For instance, the processing system may use additional heuristic rules relating to additional characteristics of the subject, or additional characteristics of the subject may be used as additional inputs to a machine-learning algorithm for determining the measure of SDB severity. The machine-learning may have been trained using input data entries that included example additional characteristics in addition to the snore rate variability characteristics.

The additional characteristics may include one or more snore energy characteristics, one or more physical characteristics, one or more demographic characteristics, one or more subjective indicators, one or more sleep quality characteristics, and/or one or more further snoring characteristics.

For example, the processing system 130 may process the first time-varying signal 125 to determine one or more snore energy characteristics, and determine the measure of SDB severity by processing at least the one or more snore rate variability characteristics and the one or more snore energy characteristics.

Snore energy characteristics are characteristics of a measure of snore energy, such as characteristics of a magnitude of snore energy and/or characteristics of a gradient of snore energy with respect to time. Various characteristics of the first time-varying signal 125, including the root mean square and amplitude, are representative of snore energy.

Methods of determining snore energy from a time-varying signal responsive to a subject's breathing are well known.

Figure 8 shows an example time-varying signal 800 for a subject during a series of hypopnea events. The time-varying signal 800 is an audio signal responsive to breathing sounds of the subject during a sleep session. As Figure 8 illustrates, the snore energy of the subject's snores has an increasing trend during each hypopnea event (characterized by the increasing amplitude of the signal 800 for successive snore events during each hypopnea event). It has been found that snore energy tends to have an increasing or decreasing trend for snoring that occurs during or close to hypopnea of apnea events, and that this is not the case for regular snoring. Characteristics of a gradient of snore energy with respect to time may therefore distinguish snoring related to sleep disordered breathing from regular snoring, and may thus be used to improve an accuracy of the determination of the measure of SDB severity.

Characteristics of a gradient of snore energy with respect to time may, for example, be determined by creating a sliding window containing a predetermined number of snores (e.g. the sliding window may contain 4 snores, with a difference of one snore between successive windows), and determining a snore energy slope for each window. A separate sliding window may be created for different periods of snoring and/or for different snore groups, where appropriate.

For instance, the snore energy slope may be determined by processing the first time-varying signal to determine the snore energy (SE) for each snore in the window and the time intervals between consecutive snores in the window. The time intervals may be used to define snore time stamps (STSs) for each snore in the window (e.g. with a snore time stamp of 0 for the first snore in the window), and the snore energies and snore time stamps may be normalized to 0 to 1. The snore energy slope (SES) for the window may then be determined by determining a set of parameters that fit equation 1:

$$SE = SES \times STS + b \tag{1}$$

Characteristics of a gradient of snore energy may include features of the snore energy slopes such as the percentage of snore energy slopes above a predetermined value, a percentile of the snore energy slopes, and statistical distribution parameters. Table V lists examples of snore energy trend features that may be used to determine the measure of SDB severity.

TABLE V

| Parameter | Unit | Definition |
|---|---|---|
| SES_ratio | % | Percentage of snore energy slopes (SES) bigger than 0.1, 0.2, 0.3, 0.4, 0.5, 0.6, 0.7, 0.8, 0.9 and 1 respectively |
| SES _percentile | | Percentile of SES at 10%, 20%, 30%, 40%, 50%, 60%, 70%, 80% and 90% respectively |
| SES_sd | | Statistical distribution parameters of SES including mean, variance, Standard deviation, skewness, kurtosis, min, max, median, and interquartile range |

Similarly, features of the snore energy during the sleep session (e.g. statistical distribution parameters, such as a mean, a variance, a standard deviation, a skewness, a kurtosis, a minimum, a maximum, a median and/or an interquartile range) may be used to determine the measure of SDB severity. Suitable features of the snore energy during the sleep session will be apparent to the skilled person.

In some examples, the one or more additional characteristics used to determine the measure of SDB severity may comprise one or more physical characteristics of the subject 110. The one or more physical characteristics may include a measure of daytime physical activity (e.g. obtained from a fitness-tracking device), a neck circumference and/or a diagnosis of a medical condition (e.g. hypertension, comorbid cardiac conditions). Further suitable physical characteristics will be apparent to the skilled person. The one or more physical characteristics may be obtained via user input (e.g. at a user input device such as the smartphone 140 in Figure 1) and/or stored in a memory unit (not shown in Figure 1).

In some examples, the one or more additional characteristics used to determine the measure of SDB severity may comprise one or more demographic characteristics of the subject 110. The one or more demographic characteristics may include an age, a body mass index (BMI) and/or a sex. Further suitable demographic characteristics will be apparent to the skilled person. The one or more physical characteristics may be obtained via user input and/or stored in a memory unit.

In some examples, the one or more additional characteristics used to determine the measure of SDB severity may comprise one or more subjective indicators obtained via user input (e.g. at a user input device such as the smartphone 140 in Figure 1). The one or more subjective indicators may include a measure of daytime sleepiness (e.g. a qualitative measure), a reported occurrence of snoring and/or a breathing pause during a previous sleep session (e.g. reported by the subject's bed partner), a reported occurrence of a difficulty initiating or maintaining sleep during a previous sleep session, a measure of stress, and/or a reported occurrence of a difficulty in concentrating. Further suitable subjective indicators will be apparent to the skilled person.

In some examples, the one or more additional characteristics used to determine the measure of SDB severity may comprise one or more sleep quality characteristics. The processing system 130 may obtain sleep data representative of a sleep quality of the subject 110 from a sleep-monitoring device (not shown in Figure 1), and process the sleep data to determine the one or more sleep quality characteristics. Data representative of a sleep quality of the subject may, for example, include movement data, heart rate data and/or respiration data. The use of sleep-monitoring devices (also known as sleep trackers) to obtain data representative of a sleep quality is well known, and suitable sleep-monitoring devices will be apparent to the skilled person.

The one or more sleep quality characteristics may comprise any suitable characteristics extracted from data obtained by a sleep monitoring device, including a total time spent sleeping, a time spent in a particular sleep stage, a percentage of the sleep session spent in a particular sleep stage, a number of sleep stage transitions during the sleep session, a rate of sleep stage transitions during the sleep session, a number of arousals during the sleep session, a rate of arousals during the sleep session, a number of awakenings during the sleep session, a rate of awakenings during the sleep session, and/or a characteristic derived from a hypnodensity. A hypnodensity is a probability distribution for each sleep stage during a sleep epoch (i.e. a short segment of the sleep session, e.g. a 30 second segment) or during the entire sleep session. Characteristics derived from a hypnodensity include an average entropy, a multiscale entropy, a general prevalence of a value, a highest achieved value, a measure of average fluctuations in value, a time until a certain percentage of a maximum value has been achieved, and any of these characteristics weighed by mean prevalence. Further suitable sleep quality characteristics will be apparent to the skilled person.

In some examples, the one or more additional characteristics used to determine the measure of SDB may comprise one or more further snoring characteristics. The processing system 130 may process the first time-varying signal 125 to determine the one or more further snoring characteristics, which may comprise at least one of: an inter event silence, a Mel-frequency cepstral coefficient (MFCC), a Mel cepstability, a running variance for one or more snore features, an apneic phase ratio, and a spectral power density (i.e. the power of the signal as a function of frequency). Further suitable snoring characteristics will be apparent to the skilled person.

An inter event silence is a total number of intervals lasting 10-60 s during which no snoring or breathing is detected. Mel-frequency cepstral coefficients (MFCCs) are well-known in the field of speech processing: see, for example, J R Deller et al. (2000), "Discrete-time processing of speech signals", New York Institute of Electrical and Electronics Engineers. A Mel cepstability is a sum of variances of a predetermined number of MFCCs related to the frames with the highest energy in each snore (e.g. the sum of variances of 12 MFCCs related to the frames (30 ms long) with the highest energy in each snore). A running variance for a snore feature is a mean value of running variances of all snore groups in the sleep session (determined by first calculating the running variance for each snore group in the sleep session and then determining the mean of the calculated running variances). The running variance may be a running variance for any suitable snore feature, including a snore energy, a snore duration, a time interval between consecutive snores, a fundamental frequency of the signal during snoring, an amplitude of the signal envelope and/or a change in any of these features. An apneic phase ratio is a relative number of snore groups with a running variance larger than a predetermined value, which may be determined using training set data. These are well-known characteristics in the field of nocturnal sound analysis: see, for example, Nir Ben-Israel et al. (2010), "Nocturnal Sound Analysis for the Diagnosis of Obstructive Sleep Apnea", 2010 Annual International Conference of the IEEE Engineering in Medicine and Biology, 6146-6149.

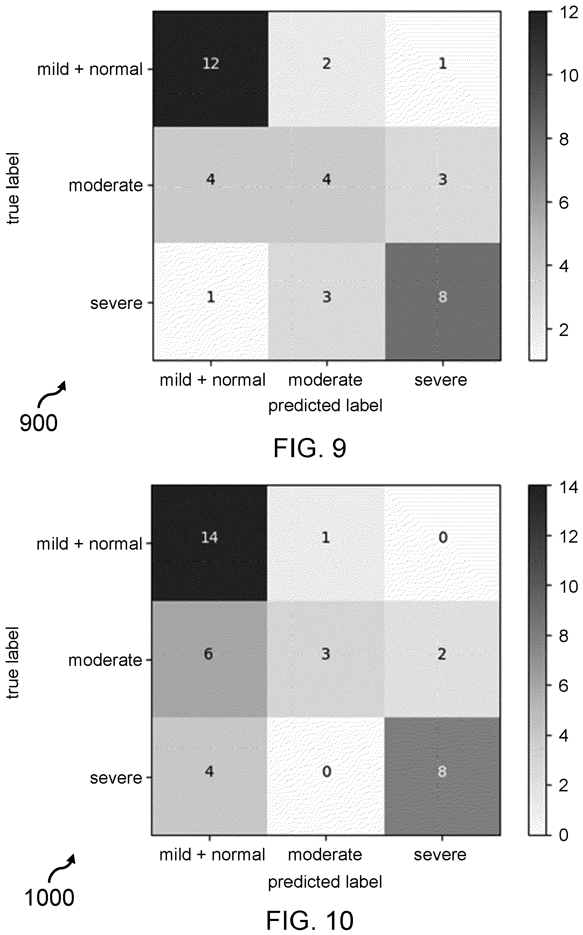

Figure 9 illustrates a confusion matrix 900 of a 5-fold cross-validation of a first trained support-vector machine (SVM) classifier with data from 38 subjects. The first SVM classifier is trained to output an estimated SDB severity level for a subject based on an input comprising snore rate variability characteristics and additional characteristics for the subject during a sleep session. The snore rate variability characteristics input to the first SVM classifier include SRV triangular index, SDSS ratio, LF relative power and LF/HF, as defined in Tables I and II. The additional characteristics input to the first SVM classifier include inter event silence, Mel cepstability, apneic phase ratio, and standard deviation of the average snore energy of each snore group during the sleep session. The output estimated SDB severity level is one of: "mild and normal" (which corresponds to an AHI below 15); "moderate" (which corresponds to an AHI between 15 and 30); and "severe" (which corresponds to an AHI above 30). The first SVM classifier has a kappa value of 0.44.

Figure 10 illustrates a confusion matrix 1000 of a 5-fold cross-validation of a second trained SVM classifier with data from the same group of subjects. The second SVM classifier is trained using the same characteristics as the first SVM classifier and a sleep quality characteristic: a percentage of the sleep session spent in N1 stage sleep. The second SVM classifier has a kappa value of 0.46, demonstrating that sleep quality characteristics may be used to further improve an accuracy of a determined measure of SDB severity.

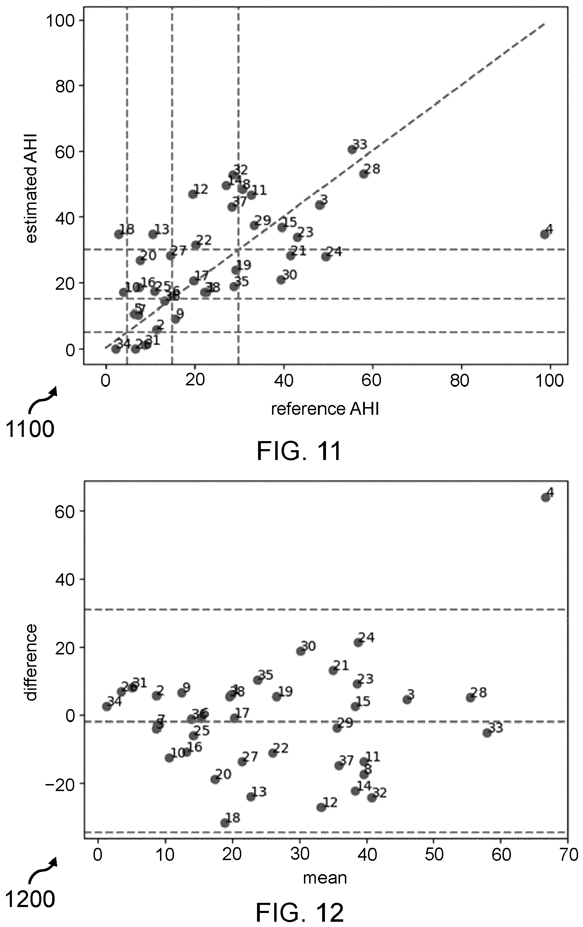

Figure 11 illustrates a scatter plot of estimated apnea hypopnea index (AHI) from a linear regression model against reference AHI from polysomnography (PSG) for the same group of subjects. Figure 12 illustrates a Bland-Altman plot of reference AHI and estimated AHI from the linear regression model. The linear regression model is trained to output an estimated AHI for a subject based on an input comprising snore rate variability characteristics and additional characteristics for the subject during a sleep session. The snore rate variability characteristics input to the linear regression model include SRV triangular index, SDSS ratio, pSS70 and TISS, as defined in Table I. The additional characteristics include inter event silence, Mel cepstability, apneic phase ratio and running variance. The linear regression model achieved a Spearman's rank correlation coefficient of 0.650 (with a P value less than 0.001).

The first and second SVM classifiers and the linear regression model are example implementations only; other machine learning models and other combinations of snore rate variability characteristics and additional characteristics are also envisaged.

Returning to Figure 1, the first breathing detector 120 may comprise a first microphone and the first time-varying signal 125 obtained by the first breathing detector may be processed to detect a first plurality of snores, as described above. In some examples, the system 100 may further comprise a second breathing detector (not shown in Figure 1) comprising a second microphone located at a different position to the first microphone. The second breathing detector may obtain a second time-varying signal responsive to the subject's breathing during the sleep session of the subject.

The processing system 130 may obtain the second time-varying signal from the second breathing detector and process the second time-varying signal to detect a second plurality of snores in the second time-varying signal, as described above with reference to the first time-varying signal.

The processing system 130 may process the first and second plurality of snores to determine a location of each snore. Methods for determining a location of an origin of a sound by detecting the sound at two different locations are very well known, so suitable methods for determining the location of each snore based on the first and second plurality of snores will be apparent to the skilled person. The determination of a location of each snore may simply comprise a determination of whether a snore originates from a desired location (e.g. using techniques such as blind source separation to isolate snores originating from the subject).

The processing system 130 may then determine time intervals only between consecutive snores that correspond to a same desired location (i.e. snores originating from the subject 110). This provides a mechanism for ensuring that only snore rate variability characteristics and additional characteristics of the subject's snores are used to determine the measure of SDB severity, and not characteristics based on snores of, for example, a bed partner of the subject.

Where two or more microphones are used to obtain time varying-signals responsive to breathing and to determine a location of each snore, the processing system 130 may additionally determine a measure of SDB severity for a second subject (e.g. a bed partner of the subject 110, or a subject in another bed in the same room as the subject 110) whose breathing sounds are also detected by the two or more microphones.

In some examples, the system 100 may comprise a third breathing detector (not shown in Figure 1), which is a different type of breathing detector to the first breathing detector. For example, the first breathing detector may comprise a microphone, while the third breathing detector comprises a nasal pressure sensor, a polvinylidene fluoride sensor, a stretchable strain sensor, an accelerometer, a gyroscope or a ballistocardiographic sensor.

The third breathing detector may obtain a third time-varying signal responsive to the subject's breathing during the sleep session of the subject. The processing system 130 may obtain the third time-varying signal from the third breathing detector, and process the third time-varying signal to detect a third plurality of snores in the third time-varying signal, as described above with reference to the first time-varying signal.

The processing system 130 may determine, for each snore in the first plurality of snores, whether the snore corresponds to a snore in the third plurality of snores. The processing system may determine that a snore in the first plurality of snore corresponds to a snore in the third plurality of snores in response to a determination that a snore in the third plurality of snores occurs within a predetermined period of the time at which the snore in the first plurality of snores occurred. The predetermined period may be a period significantly shorter than a breath cycle (e.g. assuming a shortest breath cycle of 3.33 seconds (corresponding to a breathing rate of 18 breaths per minute), the predetermined period may be a period that is much shorter than 3.33 seconds, e.g. 2 seconds or less).

The processing system 130 may then determine time intervals only between consecutive snores that each correspond to a snore in the third plurality of snores. This provides another (additional or alternative) mechanism for ensuring that only snore rate variability characteristics and additional characteristics of the subject's snores are used to determine the measure of SDB severity.

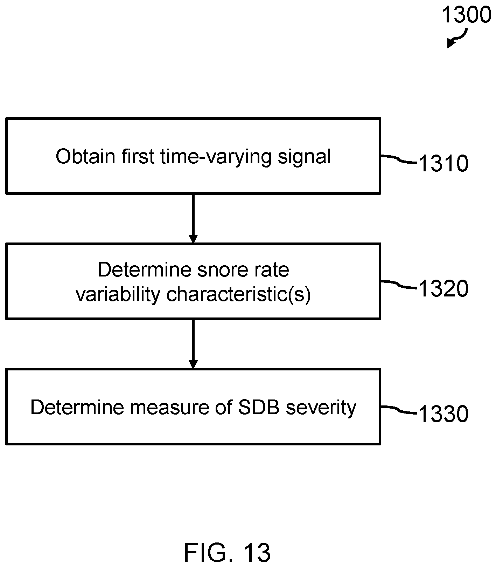

Figure 13 illustrates a computer-implemented method 1300 for determining a measure of a severity of sleep disordered breathing, according to an embodiment of the invention.

The computer-implemented method 1300 begins at step 1310, at which a first time-varying signal responsive to a subject's breathing during a sleep session is obtained from a first breathing detector.

At step 1320, the first time-varying signal is processed to determine one or more snore rate variability characteristics.

At step 1330, a measure of a severity of sleep disordered breathing is determined by processing at least the one or more snore rate variability characteristics.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A processing system for determining a measure of a severity of sleep disordered breathing, the processing system being configured to:

    obtain, from a first breathing detector, a first time-varying signal responsive to a subject's breathing during a sleep session of the subject;
    process the first time-varying signal to determine one or more snore rate variability characteristics; and
    process at least the one or more snore rate variability characteristics to determine a measure of a severity of sleep disordered breathing.

2. The processing system of claim 1, wherein the first breathing detector comprises at least one of: a microphone, a nasal pressure sensor, a polvinylidene fluoride sensor, a stretchable strain sensor, an accelerometer, a gyroscope and/or a ballistocardiographic sensor.

3. The processing system of claim 1 or 2, wherein the processing system is configured to determine the one or more snore rate variability characteristics by:

    processing the first time-varying signal to detect a first plurality of snores in the first time-varying signal;
    determining a time interval between consecutive snores in the first plurality of snores; and
    processing the time intervals between consecutive snores to determine the one or more snore rate variability characteristics.

4. The processing system of claim 3, wherein the time intervals used to determine the snore rate variability characteristics comprise only time intervals between consecutive snores that do not exceed a predetermined time interval threshold.

5. The processing system of claim 3 or 4, wherein:

the first breathing detector comprises a microphone;
the processing system is further configured to:

obtain, from a second breathing detector, a second time-varying signal responsive to a subject's breathing during a sleep session of the subject, wherein the second breathing detector comprises a microphone located at a different position to the first breathing detector;
process the second time-varying signal to detect a second plurality of snores in the second time-varying signal; and
process the first and second plurality of snores to determine a location of each snore; and

the consecutive snores between which the processing system is configured to determine a time interval are consecutive snores corresponding to a same desired location.

6. The processing system of any of claims 3 to 5, wherein:

the processing system is further configured to:

obtain, from a third breathing detector, a third time-varying signal responsive to a subject's breathing during a sleep session of the subject, wherein the first breathing detector and third breathing detector are different types of breathing detector;
process the third time-varying signal to detect a third plurality of snores in the third time-varying signal; and
for each snore in the first plurality of snores, determine whether the snore corresponds to a snore in the third plurality of snores; and

the consecutive snores between which the processing system is configured to determine a time interval are consecutive snores that each correspond to a snore in the third plurality of snores.

7. The processing system of any of claims 1 to 6, wherein the processing system is further configured to:

process the first time-varying signal to determine one or more snore energy characteristics, wherein the one or more snore energy characteristics comprise one or more characteristics of at least one measure of snore energy; and
determine the measure of the severity of sleep disordered breathing by processing at least the one or more snore rate variability characteristics and the one or more snore energy characteristics.

8. The processing system of any of claims 1 to 7, wherein the processing system is further configured to:

obtain one or more physical and/or demographic characteristics for the subject; and
determine the measure of the severity of sleep disordered breathing by processing at least the one or more snore rate variability characteristics and the one or more physical and/or demographic characteristics.

9. The processing system of any of claims 1 to 8 wherein the processing system is further configured to:

obtain, from a sleep-monitoring device, sleep data representative of a sleep quality of the sleep session of the subject;
process the sleep data to determine one or more sleep quality characteristics of the sleep session;
determine the measure of the severity of sleep disordered breathing by processing at least the one or more snore rate variability characteristics and the one or more sleep quality characteristics.

10. The processing system of any of claims 1 to 9, wherein the processing system is further configured to:

process the first time-varying signal to determine one or more further snoring characteristics, wherein the one or more further snoring characteristics comprises at least one of: an inter event silence, a Mel cepstability, a Mel-frequency cepstral coefficient, an apneic phase ratio, a spectral power density and a running variance for a time interval between consecutive snores; and

determine the measure of the severity of sleep disordered breathing by processing at least the one or more snore rate variability characteristics and the one or more further snoring characteristics.

11. The processing system of any of claims 1 to 10, wherein the processing system is configured to determine the measure of the severity of sleep disordered breathing using a machine-learning algorithm, wherein:

the machine-learning algorithm has been trained using a training algorithm configured to receive an array of training inputs and known outputs, each training input corresponding to a respective known output;
each training input comprises at least one or more snore rate variability characteristics for a sleep session for a subject; and
each known output comprises a measure of a severity of sleep disordered breathing for the subject.

12. The processing system of any of claims 1 to 11, wherein the determined measure of the severity of sleep disordered breathing is a severity level selected from a plurality of predefined severity levels, an estimated apnea hypopnea index, an estimated obstructive apnea index, an estimated hypopnea index, an estimated central apnea index, an estimated oxygen desaturation index, or an estimated respiratory disturbance index.

13. A system for determining a measure of a severity of sleep disordered breathing, the system comprising:

a breathing detector capable of obtaining a time-varying signal responsive to a subject's breathing during a sleep session of the subject; and
the processing system of any of claims 1 to 12.

14. A computer-implemented method for determining a measure of a severity of sleep disordered breathing, the computer-implemented method comprising:

obtaining, from a first breathing detector, a first time-varying signal responsive to a subject's breathing during a sleep session of the subject;
processing the first time-varying signal to determine one or more snore rate variability characteristics; and
processing at least the one or more snore rate variability characteristics to determine a measure of a severity of sleep disordered breathing.

15. A computer program product comprising computer program code means which, when executed on a computer device having a processing system, cause the processing system to perform all of the steps of the method according to claim 14.

FIG. 1

FIG. 2

FIG. 3

FIG. 4

FIG. 5

FIG. 6

FIG. 7

FIG. 8

FIG. 9

FIG. 10

FIG. 11

FIG. 12

1300

| Obtain first time-varying signal | 1310 |

| Determine snore rate variability characteristic(s) | 1320 |

| Determine measure of SDB severity | 1330 |

FIG. 13

Europäisches
Patentamt

European
Patent Office

Office européen
des brevets

# EUROPEAN SEARCH REPORT

Application Number

EP 22 18 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BEN-ISRAEL NIR ET AL: "Obstructive Apnea Hypopnea Index Estimation by Analysis of Nocturnal Snoring Signals in Adults", SLEEP, vol. 35, no. 9, 1 September 2012 (2012-09-01), pages 1299-1305, XP093001337, US ISSN: 0161-8105, DOI: 10.5665/sleep.2092 | 1-4,6-15 | INV. A61B5/48 |
| Y | * Methods p. 1299-1301 Supplemental Methods p. 1305A-1305B * | 5 | |
| X | MESQUITA J ET AL: "All night analysis of time interval between snores in subjects with sleep apnea hypopnea syndrome", MEDICAL & BIOLOGICAL ENGINEERING & COMPUTING, SPRINGER, BERLIN, DE, vol. 50, no. 4, 10 March 2012 (2012-03-10), pages 373-381, XP035037502, ISSN: 1741-0444, DOI: 10.1007/S11517-012-0885-9 | 1-4,6-15 | |
| Y | * 2. Methods p. 272-275 * | 5 | TECHNICAL FIELDS SEARCHED (IPC) |
| X | DAFNA E ET AL: "OSA severity assessment based on sleep breathing analysis using ambient microphone", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 2044-2047, XP032489031, ISSN: 1557-170X, DOI: 10.1109/EMBC.2013.6609933 [retrieved on 2013-09-25] | 1-4,6-15 | A61B G16H |
| Y | * abstract * * II. Methods p.2044-2047 * | 5 | |

-/--

The present search report has been drawn up for all claims

| Place of search | Date of completion of the search | Examiner |
|---|---|---|
| Berlin | 7 December 2022 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

# EUROPEAN SEARCH REPORT

Application Number

EP 22 18 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALENCAR ADRIANO M. ET AL: "Dynamics of snoring sounds and its connection with obstructive sleep apnea", PHYSICA A., vol. 392, no. 1, 1 January 2013 (2013-01-01), pages 271-277, XP093001772, NL ISSN: 0378-4371, DOI: 10.1016/j.physa.2012.08.008 | 1-4,6-15 | |
| Y | * 2 Methods p. 374-377 * | 5 | |
| X | WO 2012/025892 A2 (UNIV BEN GURION [IL]; MOR RESEARCH APPLIC LTD [IL] ET AL.) 1 March 2012 (2012-03-01) | 1-4,6-15 | |
| Y | * paragraphs [0041] – [0047]; figure 2 * | 5 | |
| Y | US 2008/249428 A1 (BRADLEY DONALD CARMON [CA]) 9 October 2008 (2008-10-09) * paragraphs [0005], [0006], [0032] * | 5 | |

TECHNICAL FIELDS SEARCHED (IPC)

The present search report has been drawn up for all claims

| Place of search | Date of completion of the search | Examiner |
|---|---|---|
| Berlin | 7 December 2022 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 298 989 A1**

## ANNEX TO THE EUROPEAN SEARCH REPORT
## ON EUROPEAN PATENT APPLICATION NO.

EP 22 18 2245

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2022

| Patent document cited in search report | Publication date | Patent family member(s) | Publication date |
|---|---|---|---|
| WO 2012025892 A2 | 01-03-2012 | EP 2608717 A2 | 03-07-2013 |
| | | IL 224852 A | 28-02-2017 |
| | | US 2013184601 A1 | 18-07-2013 |
| | | WO 2012025892 A2 | 01-03-2012 |
| US 2008249428 A1 | 09-10-2008 | NONE | |

For more details about this annex : see Official Journal of the European Patent Office, No. 12/82

25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIALI XIE et al.** Audio-based snore detection using deep neural networks. *Computer Methods and Programs in Biomedicine,* 2021, vol. 200, 105917
- **ELIRAN DAFNA et al.** Automatic detection of whole night snoring events using non-contact microphone. *PloS One,* 2013, vol. 8 (12), e84139
- **ERNA ARNARDOTTIR et al.** How to measure snoring? A comparison of the microphone, cannula and piezoelectric sensor. *Journal of Sleep Research,* 2016, vol. 25 (2), 158-168
- **D SANCHEZ MORILLO et al.** Monitoring and analysis of cardio respiratory and snoring signals by using an accelerometer. *Annual International Conference of the IEEE Engineering in Medicine and Biology Society,* 2007, vol. 2007, 3942-5
- **HYO-KI LEE et al.** Automatic snoring detection from nasal pressure data. *Annual International Conference of the IEEE Engineering in Medicine and Biology Society,* 2013, vol. 2013, 6870-2
- **SU HWAN HWANG et al.** Polyvinylidene fluoride sensor-based method for unconstrained snoring detection. *Physiological Measurement,* 2015, vol. 36 (7), 1399-1414
- **J R DELLER et al.** Discrete-time processing of speech signals. *New York Institute of Electrical and Electronics Engineers,* 2000
- **NIR BEN-ISRAEL et al.** Nocturnal Sound Analysis for the Diagnosis of Obstructive Sleep Apnea. *2010 Annual International Conference of the IEEE Engineering in Medicine and Biology,* 2010, 6146-6149